# EUROPÄISCHE PATENTSCHRIFT

(11) **EP 3 206 701 B1**
(45) Veröffentlichungstag und Bekanntmachung des Hinweises auf die Patenterteilung: **15.08.2018**
(21) Anmeldenummer: 15797606.9
(22) Anmeldetag: 19.10.2015
(51) Int. Cl.: A61K 36/23, A61K 36/51, A61K 36/53, A61P 13/00, A61P 13/08

(54) **CANEPHRON ZUR BEHANDLUNG VON PROSTATITIS**
CANEPHRON FOR THE TREATMENT OF ENURESIS OR PROSTATITIS
CANEPHRON DESTINÉ AU TRAITEMENT DE L'ÉNURÉSIE OU LA PROSTATITE

(30) Priorität: 17.10.2014 EP 14189410
(43) Veröffentlichungstag der Anmeldung: 23.08.2017
(73) Patentinhaber: Bionorica SE, 92318 Neumarkt (DE)
(72) Erfinder: POPP, Michael, 92318 Neumarkt (DE)
(74) Vertreter: Simandi, Claus
(86) Internationale Anmeldenummer: PCT/EP2015/074192
(87) Internationale Veröffentlichungsnummer: WO 2016/059260

(56) Entgegenhaltungen:
- PESSLER G ET AL: "Behandlung akuter und chronischer Harnwegsinfektionen mit einem Phytopharmakon = Treatment of acute and chronic urinary tract infections with a plant product", THERAPIEWOCHE, KARLSRUHE, DE, Bd. 29, Nr. 14, 1. Januar 1979 (1979-01-01), XP009178965, ISSN: 0040-5973
- CTM ET AL: "Conservative treatment of vesicoureteral reflux in a child 10 years", SOVREMENNYE TEHNOLOGII V MEDICINE 2013 NIZHNY NOVGOROD STATE MEDICAL ACADEMYRUS, Bd. 5, Nr. 3, 1. Januar 2013 (2013-01-01), Seiten 119-120, XP055158854, ISSN: 2076-4243
- "Phytokombination beugt Rezidiven vor /// Urinary tract infections. Combination phytotherapy prevents recurrences", FORTSCHRITT DER MEDIZIN MMW ORIGINALIA, URBAN UND VOGEL MEDIEN UND MEDIZIN VERLAGSGESELLSCHAFT, DE, Bd. 155, Nr. 6, 4. April 2013 (2013-04-04) , Seiten 62-63, XP009179051, ISSN: 1438-3276

## Beschreibung

Die Erfindung betrifft ein Arzneimittel zur Behandlung von Entzündungserkrankungen der Prostata, insbesondere Prostatitis als auch ein Nahrungsergänzungsmittel sowie deren Verwendung, insbesondere als Phytopharmaka, welche Pflanzen(drogen) enthalten, wobei die Pflanzen ausgewählt sind aus der Gruppe bestehend aus: Rosmarin (Rosmarinus officinalis/Rosmarini folium), Liebstöckel (Levisticum officinale/Levistici radix) und Tausendgüldenkraut (Centaurium erythraea/Centaurii herba) sowie deren Mischungen.

Die Kombination der genannten Heilpflanzen ist zur Behandlung von Harnwegserkrankungen, insbesondere Blasenentzündung unter der für die Anmelderin eingetragenen Marke Canephron®N bekannt und seit Jahrzehnten auf dem Markt. Die in Canephron®N verwendeten Heilpflanzen werden gezielt ausgewählt, untersucht und weiterverarbeitet. Die hierdurch erzielte gleich bleibende Qualität des Arzneimittels erreicht der Hersteller, die Firma BIONORICA®, durch optimierte Anzucht- und Erntestrategien sowie einer strengen Qualitätskontrolle.

Jede Einzeldroge trägt dabei einen Anteil zur einzigartigen Wirksamkeit der Zusammensetzung bei:
Rosmarin (Rosmarinus officinalis, Rosmarini folium, Synonyme: R. angustifolius, R. flexuosus, R. latifolius, R. laxiflorus, Salvia rosmarinus) wird in der Naturheilkunde seit jeher zur Anregung des Gallen- und Harnflusses und zur Entblähung verwendet.

Wirkstoffe aus der Liebstöckelwurzel (Levisticum officinale / Levistici radix, Synonyme: Angelica levisticum ALL., Angelica paludapifolia LAM., Hipposelinum levisticum BRITTON et ROSE, Levisticum levisticum KARSTEN, Levisticum paludapifolium ASCHERS, Levisticum vulgare RCHB., Ligusticum levisticum L., Ligusticum officinale PILGER) werden vor allem wegen ihres harntreibenden und krampflösenden Effekts geschätzt.

Tausendgüldenkraut (Centaurium erythraea, Synonyme: Centaurii herba, Centaurium minus MOENCH, Centaurium umbellatum GILIBERT, Erythraea centaurium (L.) PERSOON) findet wegen seiner harntreibenden, desinfizierenden und entzündungshemmenden Wirkung traditionell bei Erkrankungen des Harntraktes Anwendung.

Die gezielte Kombination aus Rosmarinblättern, Liebstöckelwurzel und Tausendgüldenkraut wirkt zugleich antibakteriell, durchspülend, entzündungshemmend sowie entspannend (spasmolytisch) auf die Harnblase. Es entsteht ein einzigartig breites Wirkungsspektrum bestehend aus antioxidativer, anti-adhäsiver, spasmolytischer, schmerzlindernder und nephroprotektiver Wirkung.

Die dem Canephron®N zugrunde liegende Zusammensetzung ist daher vorzugsweise bei entzündlichen Erkrankungen und Infektionen der ableitenden Harnwege, weiterhin zur Durchspülung der Harnwege zur Verminderung der Ablagerung von Nierengrieß und Blasensteinen geeignet und zugelassen. Canephron®N ist beispielsweise in Apotheken (Deutschland, Österreich, u.a.) erhältlich.

Canephron®N wird bei der Behandlung von durch harnwegsrelevante Erreger ausgelösten Infektionen eingesetzt. Die entzündungshemmende Wirkung wird durch die zusätzliche spasmolytische Wirkung ergänzt.
Es besteht jedoch ein großes Bedürfnis das Spektrum des potenten Arzneimittels Canephron®N zu erweitern und neue Indikationen zu erschließen und hierzu entsprechende Arzneimittel bereitzustellen.
Ausgehend vom diesem Stand der Technik ist es daher Aufgabe der vorliegenden Erfindung, neue Indikationen für ein Arzneimittel zu erschließen, wobei die Pflanzen ausgewählt sind aus der Gruppe bestehend aus: Rosmarin (Rosmarinus officinalis/Rosmarini folium), Liebstöckel (Levisticum officinale/Levistici radix) und Tausendgüldenkraut (Centaurium erythraea/Centaurii herba).
Überraschender Weise konnte für eine Zusammensetzung aus Rosmarin (Rosmarinus officinalis/Rosmarini folium), Liebstöckel (Levisticum officinale/Levistici radix) und Tausendgüldenkraut (Centaurium erythraea/Centaurii herba) gefunden werden, dass sie für Entzündungserkrankungen der Prostata, insbesondere Prostatitis, ebenfalls geeignet ist.
Daher betrifft die Erfindung ein Mittel, insbesondere Arzneimittel oder Nahrungsergänzungsmittel, umfassend Rosmarin (Rosmarinus officinalis/Rosmarini folium), Liebstöckel (Levisticum officinale/Levistici radix) und Tausendgüldenkraut (Centaurium erythraea/Centaurii herba) zur Verwendung oder Anwendung, insbesondere in der Prophylaxe und Behandlung von Prostataentzündungserkrankungen, insbesondere Prostatitis.

In einer weiteren bevorzugten Ausführungsform des erfindungsgemäßen Mittels ist das Verhältnis von Rosmarin (Rosmarinus officinalis/Rosmarini folium), Liebstöckel (Levisticum officinale/Levistici radix) und Tausendgüldenkraut ((Centaurium erythraea/Centaurii herba) 1 : 1 : 1, jeweils +/-0,2 bis 0,4 (wie z.B. 1 : 1,2 : 0,6 oder 1 : 1,4 : 1,1). Erfindungsgemäß umfasst sind sämtliche Pflanzenteile, jedoch im Fall von Rosmarin sind die Blätter bevorzugt, im Fall von Liebstöckel ist die Wurzel bevorzugt, im Fall von Tausendgüldenkraut das erdüberstehende Kraut. Ferner sind die Arten Rosmarinus officinalis (Rosmarini folium), Levisticum officinale (Levistici radix) und Centaurium erythraea (Centaurii herba) bevorzugt, jedoch sind ebenfalls alle andere Arten der Gattungen Rosmarinus, Levisticum und Centaurium erfindungsgemäß umfasst.
Weiterhin ist bevorzugt, dass die jeweils vorgelegte Pflanzen(droge) gereinigt und geschnitten ist und anschließend gemischt werden. Die Reinigung mittels 96% -igen Ethanol und Wasser ist bevorzugt.
Die derart erhaltenen Pflanzen(drogen) werden zu pharmazeutischen Zubereitungen weiter verarbeitet.
Im Rahmen dieser Erfindung wird unter Prostataentzündung(serkrankungen) solche verstanden, wie nicht abschließend Prostatitis. Weiterhin kann "Prostatitis" differenziert werden, in: "akute Prostatitis" (Kategorie I), "chronische bakterielle Prostatitis" (Kategorie II), "chronische abakterielle Prostatitis" / "chronisches Schmerzsyndrom des Beckens" (Kategorie III), "asymptomatische entzündliche Prostatitis" (Kategorie IV), wobei alle diese Formen erfindungsgemäß umfasst sind. Prostatitis kann in eine akute oder chronische unspezifische Entzündung der Prostata durch urogene oder hämatogene Infektion oder mittels Übergreifen einer Entzündung der Nachbarorgane entstehen.

Eine Prostatitis ist meist mit Schmerzen und hohem Beschwerdedruck begleitet, wie Algurie (Schmerzen und Brennen beim Wasserlassen), Schmerzen in der Penis-, Hoden-, Damm-, Anal-, Leisten-, Scham- sowie in der Lendengegend und Schmerzen während und insbesondere nach der Ejakulation.

Das erfindungsgemäße Mittel führt insbesondere wirksam zur Reduktion von pro-inflammatorischen Zytokinen, die bei Prostatitis am Entzündungsprozess beteiligt sind (siehe Beispiel 3). Ferner konnte im Tierexperiment dargelegt werden, dass das erfindungsgemäße Mittel wirksam in der Lage ist, Ödembildung als Teil einer Entzündungsreaktion zu verhindern (siehe Beispiel 4). Schließlich verhindert das erfindungsgemäße Mittel eine Erhöhung der Schmerzempfindlichkeit, wie sie als Folge einer Prostatitis bzw. der zugrundeliegenden Entzündungsreaktion spezifisch auftritt (siehe Beispiel 5). Folglich wirkt das erfindungsgemäße Mittel vorteilhaft schmerzlindernd auch unter Minderung des Beschwerdedruckes, insbesondere im Fall einer Prostatitis.

Das erfindungsgemäße Mittel führt weiterhin wirksam zur Entspannung kontrahierter Blasenstreifen und wirkt daher einer unkontrollierten Miktion entgegen (siehe Beispiel 1). Im Tierexperiment kann gezeigt werden, dass die Behandlung mit dem erfindungsgemäßen Mittel zu einer Verringerung der Miktionsanzahl in einem gegebenen Zeitraum führt (siehe Beispiel 2). Dies spricht dafür, dass das wirkende Mittel nicht nur geeignet ist einer Drangharnsymptomatik entgegen zu wirken, sondern darüber hinaus vorteilhaft in der Lage ist, vorzeitige Miktionen - wie z.B. bei einer Enuresis gegeben - zu verhindern.
Das wirkende Mittel, enthaltend die erfindungsgemäßen Pflanzen ausgewählt aus der Gruppe bestehend aus: Rosmarin (Rosmarinus officinalis/Rosmarini folium), Liebstöckel (Levisticum officinale/Levistici radix) und Tausendgüldenkraut (Centaurium erythraea/Centaurii herba) (nachstehend: erfindungsgemäßes Mittel), kann nunmehr vorteilhaft zur Behandlung und Prophylaxe eines erkrankten Patienten oder Individuums, Tier, Säugetier oder vorzugsweise Mensch und zwar zur Behandlung und Prophylaxe von Prostataentzündungserkrankungen, insbesondere Prostatitis, ohne den Einsatz von synthetisch-chemischen Komponenten eingesetzt werden. Auf diese Weise kann der Einsatz von Antibiotika vorteilhaft vermieden werden.
Die galenische Formulierung des erfindungsgemäßen Mittels kann ausgewählt sein aus der Gruppe bestehend aus: Tropfen, Saft, Sirup, Tabletten, Dragees, Kapseln, Retard-Formulierungen, Rektal- oder Vaginalsuppositorien, Infusionen, Aufguss, Desinfektionslösungen, Salben, Emulsionen, Puder, Pulver, flüssige oder feste Präparate für die Inhalation, Kompressen, Einlagen, Tamponaden. Selbstverständlich kann die Formulierung pharmazeutisch übliche Hilfsstoffe enthalten.

In einer weiteren Ausführungsform betrifft die Erfindung ein Arzneimittel enthaltend ein erfindungsgemäßes Mittel zur Verwendung oder Anwendung zur Behandlung und Prophylaxe von Prostataentzündungserkrankungen, insbesondere Prostatitis. Weiterhin betrifft die Erfindung bevorzugt ein Arzneimittel enthaltend ein erfindungsgemäßes Mittel zur Verwendung oder Anwendung zur Behandlung und Prophylaxe von akuter Prostatitis, chronische bakterielle Prostatitis, chronische abakterielle Prostatitis, asymptomatische entzündliche Prostatitis.
Eine weitere bevorzugte Ausführungsform betrifft ein Nahrungsergänzungsmittel enthaltend das erfindungsgemäße Mittel, insbesondere in Form einer diätischen Zusammensetzung. Geeignete erfindungsgemäße Nahrungs- oder Lebensmittel, einschließlich Wasser, sind solche wie z.B. in der Verordnung (EG) Nr. 178/2002 vom 28. Januar 2002 nicht abschließend definiert, solche wie Backwaren und Getränke und Kindernahrungszubereitungen. Das erfindungsgemäße Nahrungsergänzungsmittel kann mit einem geeigneten physiologisch verträglichen Träger versetzt werden.
Die erfindungsgemäßen pharmazeutischen Zubereitungen können in Form von Dosierungseinheiten hergestellt werden. Dies bedeutet, dass die Zubereitungen in Form einzelner Teile, vorzugsweise Kapseln und Ampullen vorliegen, deren Wirkstoffgehalt an Pflanzentrockenextrakten einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge des erfindungsgemäßen Mittels, die bei einer Applikation verabreicht wird und die gewöhnlich einer ganzen, einer halben, einem Drittel oder einem Viertel einer Tagesdosis entspricht. Bevorzugt ist eine Dosierung von dreimal täglich, vorzugsweise in Form einer Tablette oder Tropfen, insbesondere morgens, mittags und abends, ggfs. zu den Mahlzeiten.

In einer weiteren bevorzugten Ausführungsform kann die galenische Formulierung einer Kalziummanteltablette gewählt werden, wie in EP 1392337 offenbart.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen, wie a) Füll- und Streckmittel, z.B. Stärken, Milchzucker, Rohrzucker, Glukose, Mannit, Dextrine, Maltodextrin und Kieselsäure, hochdisperses Siliciumdioxid, b) Bindemittel, z.B. Carboxymethylcellulose, Cellulosepulver, mikrokristalline Cellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein, z.B. solche wie nicht abschließend Hypromellose, mikrokristalline Cellulose, Stearinsäure, Titandioxid, und ebenfalls so zusammengesetzt sein, dass sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können. Daher betrifft die Erfindung ebenfalls eine pharmazeutische Zubereitung enthaltend ein erfindungsgemäßes Mittel samt Hilfs- und Zusatzstoffen.

### Beispiele und Figuren:

Diese Beispiele und Figuren dienen ausschließlich zur Erläuterung der Erfindung. Canephron®N wurde für orale Gabe in Wasser oder 50% Ethanol angesetzt. Für *in vitro* Experimente wurde Cabnephron®N in 50% Ethanol gelöst und Verdünnungen in den Experimenten eingesetzt.

### Beispiel 1:

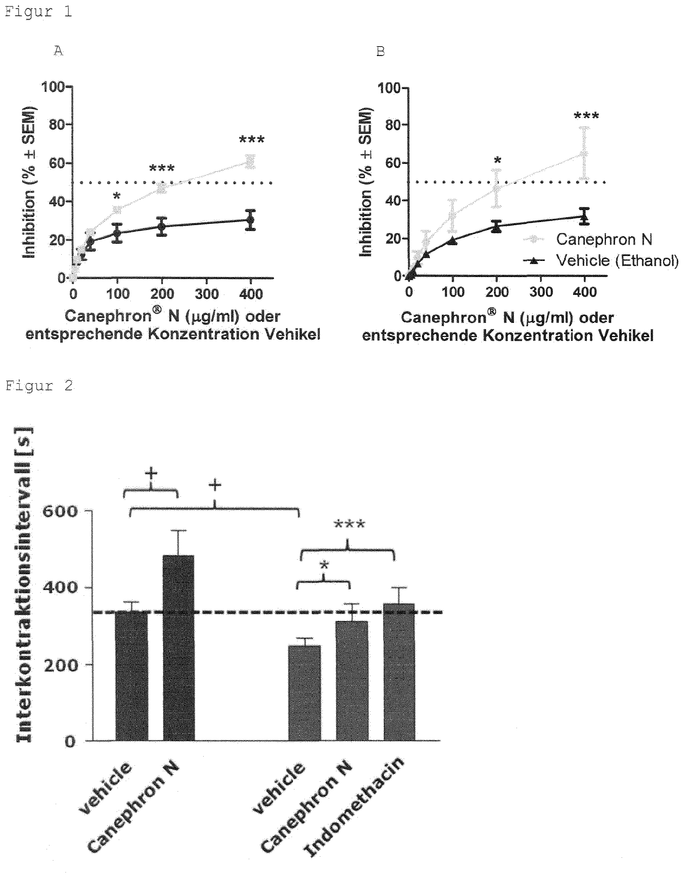

Spasmolytische Wirkung: Canephron®N wirkte spasmolytisch auf Gewebestreifen der Rattenblase (Figur 1A) und menschlichen Blase (Figur B). Gewebestreifen der Rattenblase oder menschlichen Blase wurden in einem Organbad an einem Kraftmesser fixiert und mit Kaliumchloridlösung kontrahiert. Nach Zugabe steigender Konzentrationen an Canephron®N oder Vehikel wurde die spasmolytische Wirkung gemessen. Im Vergleich zu der entsprechenden Konzentration an Vehikel bewirkte Canephron®N eine signifikant stärkere Entspannung (Inhibition). Übertragen deutet diese Entspannung der Gewebestreifen auf eine Verbesserung der Blasenkapazität und somit eine Verringerung der Häufigkeit der Blasenentleerung hin.

### Beispiel 2:

Spasmolytische Wirkung: Canephron®N reduzierte in der Ratte die Häufigkeit der Blasenentleerung *in vivo.*

Canephron®N (666 mg/kg) oder Vehikel (Wasser) wurde oral verabreicht. Anschließend wurde ein Katheter in die Blase implantiert, um diese mit konstanter Rate (50 µl/min) zu füllen und den intravesikalen Druck zu messen.

Während der Stabilisationsperiode wurde die Frequenz der Blasenentleerung gemessen. In dieser Periode erhöhte Canephron®N das Intervall zwischen zwei Blasenentleerungen (Figur 2, Interkontraktionsintervall, linke Balken), d.h. reduzierte die Häufigkeit der Blasenentleerung in nichtirritierter Blase.

Im Anschluss an die Stabilisationsperiode wurde die Blase durch Instillation von Essigsäure irritiert und erneut die Häufigkeit der Blasenentleerung gemessen. Auch bei irritierter Blase mit erhöhter Häufigkeit der Blasenentleerung (reduziertes Intervall) reduzierte Canephron®N die Häufgkeit der Blasenentleerung (d.h. erhöhte das Interkontraktionsinterval, Figur 2, rechte Balken).

### Beispiel 3:

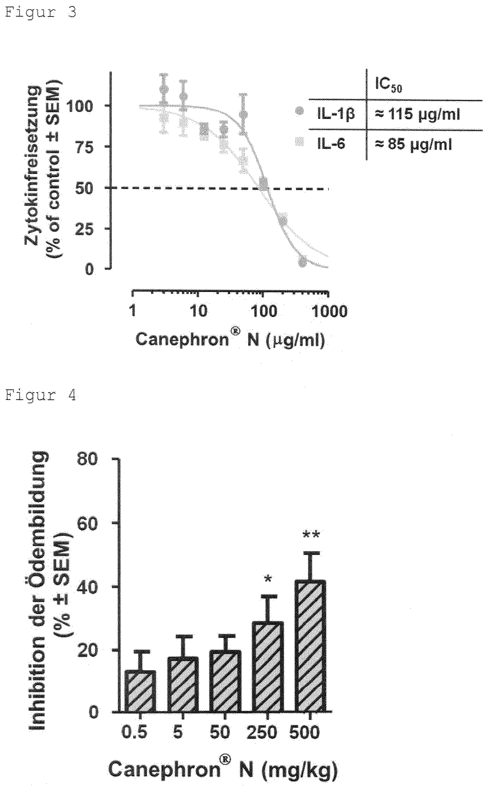

Anti-entzündliche Wirkung: Canephron®N reduzierte die Freisetzung der entzündungsfördernden Zytokine IL-1β und IL-6:
Mausmakrophagen wurden mit ansteigenden Konzentrationen an Canephron®N oder Vehikel inkubiert und anschließend durch Zymosan zur Freisetzung der Zytokine 1L-1β und IL-6 stimuliert. Die Konzentration dieser Zytokine wurde mit einem Multiplex-Assay gemessen. Die anti-entzündliche Wirkung von Canephron®N ist ersichtlich an der konzentrationsabhängigen Inhibition der Zytokinfreisetzung (Figur 3).

### Beispiel 4:

Anti-entzündliche Wirkung: Canephron®N reduzierte die Ödembildung in der Ratte *in vivo.*

Nach oraler Gabe von Canephron®N wurde durch Injektion von Carrageenan in die Hinterpfote eine Entzündung und eine damit einhergehende Schwellung (Ödem) ausgelöst. Das Volumen der Schwellung wurde als Maß der Entzündung durch Wasserverdrängung (Plethysmographie) bestimmt. Canephron®N reduzierte konzentrationsabhängig die Entstehung des Ödems (Figur 4). Diese *in vivo* Daten zeigen anti-entzündliche und abschwellende Wirkungen von Canephron®N, und sind ein deutlicher Hinweis auf die Wirksamkeit von Canephron®N bei der Behandlung von Prostatitis.

### Beispiel 5:

Schmerzlindernde Wirkung: Canephron®N normalisierte die Schmerzschwelle in experimenteller Prostatitis in der Ratte *in vivo.*

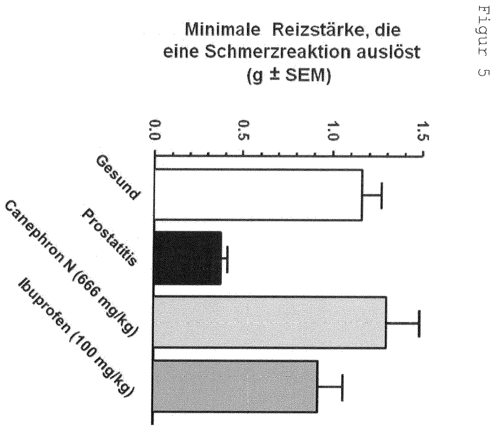

Nach Gabe von Canephron®N oder Vehikel wurde durch eine Injektion von Carrageenan eine experimentelle Prostatitis ausgelöst (Figur 5). Am zweiten Tag nach Induktion der Prostatitis wurde die Schmerzschwelle ermittelt. Dazu wurden von Frey Filamente von zunehmender Stärke verwendet und die minimale Stärke, die eine Schmerzreaktion auslöste (Schmerzschwelle) ermittelt. Canephron®N normalisierte die Schmerzschwelle in experimenteller Prostatitis, wie auch im Vergleich zum entzündungshemmenden Medikament Ibuprofen®, und ist somit ein vielversprechendes Medikament für die Behandlung von Prostatitis.

## Patentansprüche

1. Arzneimittel oder Nahrungsergänzungsmittel umfassend Rosmarin (Rosmarinus officinalis/Rosmarini folium), Liebstöckel (Levisticum officinale/Levistici radix) und Tausendgüldenkraut (Centaurium erythraea/Centaurii herba) zur Verwendung in der Prophylaxe und Behandlung von Prostataentzündungserkrankungen.

2. Arzneimittel oder Nahrungsergänzungsmittel zur Verwendung nach Anspruch 1 in der Prophylaxe und Behandlung von Prostatitis.

3. Arzneimittel oder Nahrungsergänzungsmittel zur Verwendung nach Anspruch 1 in der Prophylaxe und Behandlung von Prostatitis, akuter Prostatitis, chronische bakterielle Prostatitis, chronische abakterielle Prostatitis, asymptomatische entzündliche Prostatitis.

4. Arzneimittel oder Nahrungsergänzungsmittel zur Verwendung nach einem der Ansprüche 1 bis 3, wobei das Verhältnis von Rosmarin (Rosmarinus), Liebstöckel (Levisticum) und Tausendgüldenkraut (Centaurium) 1:1:1, jeweils +/- 0,2 bis 0,4 beträgt.

5. Pharmazeutische Zubereitungen enthaltend ein Mittel zur Verwendung nach einem der Ansprüche 1 bis 4, ggfs. samt geeigneter Trägerstoffe, insbesondere in Form von Tropfen, Saft, Sirup, Tabletten, Dragees, Kapseln, Retard-Formulierungen, Rektal- oder Vaginalsuppositorien, Infusionen, Aufguss, Desinfektionslösungen, Salben, Emulsionen, Puder, Pulver, flüssige oder feste Präparate für die Inhalation, Kompressen, Einlagen, Tamponaden.

6. Pharmazeutische Zubereitungen enthaltend ein Mittel zur Verwendung nach einem der Ansprüche 1 bis 4 samt Hilfs- und Zusatzstoffen.

## Claims

1. Medicinal product or dietary supplement comprising rosemary (Rosmarinus officinalis/Rosmarini folium), lovage (Levisticum officinale/Levistici radix) and lesser centaury (Centaurium erythraea/Centaurii herba) for use in the prophylaxis and treatment of inflammatory diseases of the prostate.

2. Medicinal product or dietary supplement for use according to claim 1 in the prophylaxis and treatment of prostatitis.

3. Medicinal product or dietary supplement for use according to claim 1 in the prophylaxis and treatment of prostatitis, acute prostatitis, chronic bacterial prostatitis, chronic abacterial prostatitis, or asymptomatic inflammatory prostatitis.

4. Medicinal product or dietary supplement for use according to any one of claims 1 to 3, wherein the ratio of rosemary (Rosmarinus), lovage (Levisticum) and lesser centaury (Centaurium) is 1:1:1, in each case +/- 0.2 to 0.4.

5. Pharmaceutical preparations containing an agent for use according to any one of claims 1 to 4, optionally together with suitable carrier substances, in particular in the form of drops, juice, syrup, tablets, lozenges, capsules, slow-release formulations, rectal or vaginal suppositories, infusions, vaporisations, disinfectant solutions, ointments, emulsions, particulate material, powders, liquid or solid preparations for inhalation, compresses, wadding, tamponades.

6. Pharmaceutical preparations containing an agent for use according to any one of claims 1 to 4 together with auxiliaries and additives.

## Revendications

1. Produit pharmaceutique ou produit de supplémentation alimentaire comprenant du romarin (Rosmarinus officinalis/Rosmarini folium), de la livèche (Levisticum officinale/Levistici radix) et de la petite centaurée (Centaurium erythraea/Centaurii herba) pour un emploi dans la prophylaxie et le traitement de maladies inflammatoires de la prostate.

2. Produit pharmaceutique ou produit de supplémentation alimentaire pour un emploi selon la revendication 1 dans la prophylaxie et le traitement de la prostatite.

3. Produit pharmaceutique ou produit de supplémentation alimentaire pour un emploi selon la revendication 1 dans la prophylaxie et le traitement de la prostatite, de la prostatite aigue, de la prostatite bactérienne chronique, de la prostatite abactérienne chronique, de la prostatite inflammatoire asymptomatique.

4. Produit pharmaceutique ou produit de supplémentation alimentaire pour un emploi selon l'une des revendications 1 à 3, où le rapport de romarin (Rosmarinus), de livèche (Levisticum) et de petite centaurée (Centaurium) est de 1 : 1 : 1, à respectivement +/- 0,2 à 0,4 près.

5. Préparations pharmaceutiques contenant un produit pour un emploi selon l'une des revendications 1 à 4, avec éventuellement des supports appropriés, en particulier sous la forme de gouttes, de jus, de sirop, de comprimés, de dragées, de gélules, de formulations à libération différée, de suppositoires rectaux ou vaginaux, d'infusions, de décoctions, de solutions désinfectantes, de pommades, d'émulsions, de poudre, de produit pulvérisé, de préparations liquides ou solides pour l'inhalation, de compresses, de dépôts, de tamponnades.

6. Préparations pharmaceutiques contenant un produit pour un emploi selon l'une des revendications 1 à 4 y compris les produits auxiliaires et les additifs.
